(19) Europäisches Patentamt
European Patent Office
Office européen des brevets

(11) Publication number: 0 341 884
A1

# (12) EUROPEAN PATENT APPLICATION

(21) Application number: 89304396.8

(22) Date of filing: 02.05.89

(51) Int. Cl.4: C12Q 1/28 , G03C 7/30 , G01N 33/52

(30) Priority: 02.05.88 US 188998

(43) Date of publication of application: 15.11.89 Bulletin 89/46

(84) Designated Contracting States: AT BE CH DE ES FR GB GR IT LI LU NL SE

(71) Applicant: EASTMAN KODAK COMPANY (a New Jersey corporation)
343 State Street
Rochester New York 14650(US)

(72) Inventor: Belly, Robert Troconis c/o Eastman Kodak Company
Patent Department 343 State Street
Rochester New York 14650(US)
Inventor: Michno, Drake Matthew c/o Eastman Kodak Company
Patent Department 343 State Street
Rochester New York 14650(US)
Inventor: Fleckenstein, Lee Joseph c/o Eastman Kodak Company
Patent Department 343 State Street
Rochester New York 14650(US)
Inventor: Washburn, William N. c/o Eastman Kodak Company
Patent Department 343 State Street
Rochester New York 14650(US)

(74) Representative: Nunney, Ronald Frederick Adolphe et al
Kodak Limited Patent Department Headstone Drive
Harrow Middlesex HA1 4TY(GB)

## (54) Novel reagent and method for assaying hydrogen peroxide.

(57) Reagents for assaying hydrogen peroxide are disclosed which comprise

a) a material having peroxidative activity;

b) a hydrogen donating primary amine; and

c) a compound selected from those having the general formula:

$COUP\text{-}(LINK)_n\text{-}R$

wherein

COUP- represents a radical that couples with an oxidized primary amine and releases -(LINK)$_n$-R;

-LINK- represents a divalent radical that undergoes intramolecular cyclization and release of -R upon release by COUP-;

n represents zero or one;

-R represents a monovalent radical that forms a detectable species in the form of a colorimetric dye or fluorescent compound upon release from -LINK- or COUP-.

EP 0 341 884 A1

# NOVEL REAGENT AND METHOD FOR ASSAYING HYDROGEN PEROXIDE

The present invention relates to novel reagents, methods and elements for clinical chemistry.

Dye forming reactions are widely used in clinical chemistry assays. An example includes assays for hydrogen peroxide ($H_2O_2$) and analytes which generate hydrogen peroxide. Analytes of interest include glucose, cholesterol, uric acid, etc.

Assays for the presence of hydrogen peroxide are carried out in the presence of a material having peroxidate activity, such as peroxidase. Other reagents involved in the test are a hydrogen donating primary amine and a coupler. For example, EPO 0 060 518 discloses a method comprising reacting a sample containing hydrogen peroxide with a reagent comprising a hydrogen donating primary amine, peroxidase and a compound having the formula Q-fl. Q is derived from photographic couplers and fl is a fluorescent coumarin derivative. The assay is carried out by combining an unknown sample with the reagent. The amine is oxidized by $H_2O_2$ in the presence of peroxidase. The oxidized amine reacts with Q-fl to release fl.

The foregoing method for detecting hydrogen peroxide is inadequate because coumarin dyes have high pKa values and therefore have limited usefulness throughout the physiological pH range (6 to 9). Coumarins also have absorptions below 500 nm where many serum interferents are active.

The present invention provides a reagent comprising

a) a material having peroxidative activity; and

b) a hydrogen donating primary amine characterized in that the reagent also includes

c) a compound selected from those having the general formula:

$COUP\{LINK\}_{n}R$

wherein

COUP- represents a radical that couples with an oxidized primary amine and releases -(LINK)$_n$-R;

-LINK- represents a divalent radical that undergoes intramolecular cyclization and release of -R upon release by COUP-;

n represents zero or one;

-R represents a monovalent radical that forms a detectable species in the form of a colorimetric dye or fluorescent compound upon release from -LINK- or COUP-;

wherein -R is selected from the group consisting of:

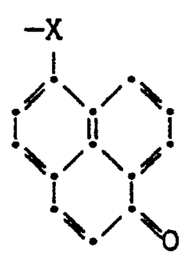 ; 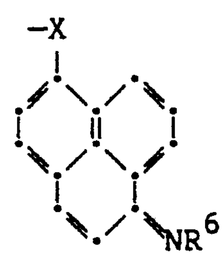 ;

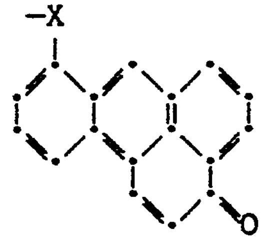 ; 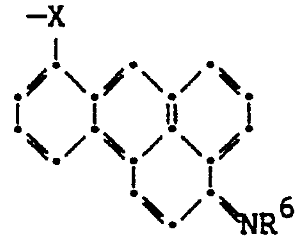 ;

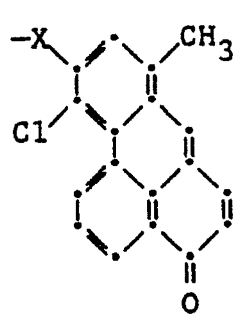 ; 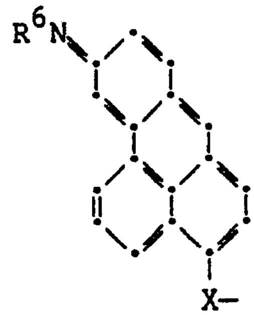 ;

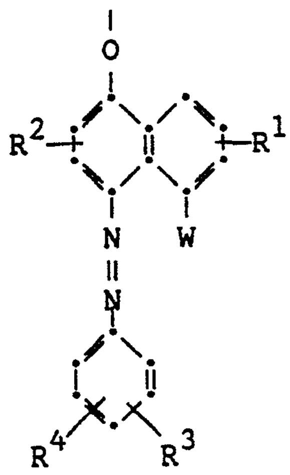 ;

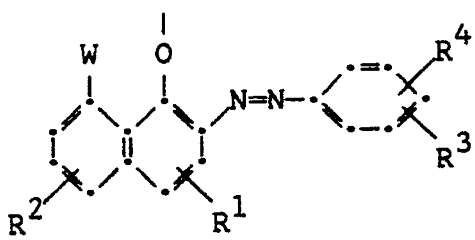 ;

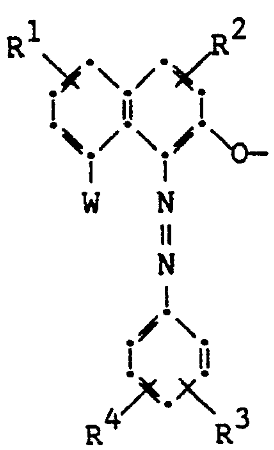 ; wherein

W represents hydrogen, halogen such as chloro or bromo; hydroxy; substituted or unsubstituted carbonamido such as acetamido; sulfonamido; phenylsulfonyl; ureido or amino;

$R^1$ and $R^2$ each independently represent hydrogen; halogen; alkyl such as methyl, ethyl, propyl; alkoxy such as methoxy, t-butoxy; carboxy; sulfo; cyano; nitro; carboxylic acid ester; benzoyl; carbonamido; sulfonamido; alkylsulfonyl such as methanesulfonyl; or arylsulfonyl such as benzenesulfonyl; and

$R^3$ and $R^4$ each independently represent halogen; nitro; sulfonamido; carbonamido; benzoyl; cyano; alkylsulfonyl such as methane- sulfonyl; or arylsulfonyl such as benzenesulfonyl;

$R^5$ represents H; alkyl such as methyl; ethyl or butyl; cycloalkyl such as cyclohexyl; or aryl such as phenyl or napthyl;

$R^6$ represents H; $CH_3$ or $C_2H_5$; and

X represents -O-; -S- or

$-\overset{|}{N}R^5$.

The compounds $COUP\{LINK\}_n R$ combine with the amine and the peroxidative material, in the presence of $H_2O_2$, to release a detectable species in the form of 1) colorimetric dyes having high extinction and absorption at wavelengths greater than 500 nm or 2) fluorescent dyes having absorptions and emissions above 500 nm and low pKa values. They are therefore useful in the physiological pH range.

The compounds, covered by the general formula $COUP\{LINK\}R$, fall into two general groups.

Group I are those in which n represents zero. In these compounds -R is linked directly to COUP- without the intervening linking group -LINK-. -R is directly released in the presence of an oxidized amine and forms the detectable species.

Group II include those compounds in which n equals 1. These compounds are anchimeric releasing couplers. This means that in the presence of an oxidized amine, the -LINK-R portion is released from COUP- and undergoes intramolecular reaction to form a heterocyclic ring followed by release of -R to form the detectable species.

In both groups of compounds, couplers are known in the photographic arts from which the COUP- component may be easily made. COUP- radicals are disclosed, for example in European Patent Application 0 060 518; U.S. Patent 3,443,940 and U.S. Patent 3,148,062.

The compounds of group I are formed using such known couplers by reacting the latter with compound containing -R. In general, the reaction is carried out using any of the techniques known in the photographic art for forming dye releasing couplers. Such methods are exemplified for example, in European Patent Application 0 060 518.

The compounds of group II include the linking group -LINK- between the -R and COUP-. The compounds of this group utilize the same COUP-radicals used in group I. Examples of linking compounds include:

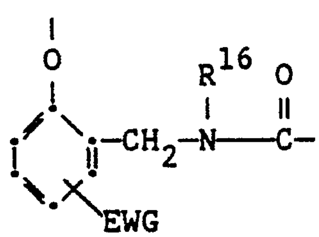 ;

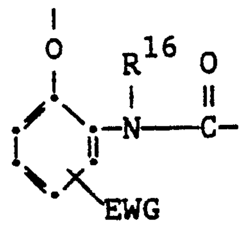

;

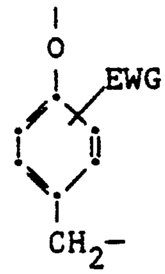

;

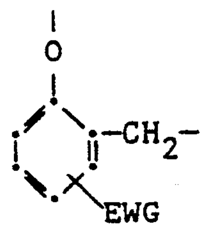

; wherein

$R^{16}$ represents $CH_3$, $C_2H_5$, n-$C_3H_7$ or i-$C_3H_7$;
EWG represents an electron withdrawing group in ortho or para position relative to the oxy group -O-, such as $-NO_2$, $-CO_2R^{17}$, $-SO_2R^{17}$, $-SO_2NR_2^{17}$ or -CN,
wherein $-R^{17}$ represents H, alkyl such as methyl, ethyl or octadecyl; or aryl such as phenyl, tolyl or napthyl.

Preferred examples of -LINK- are:

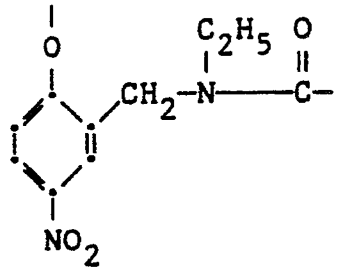

;

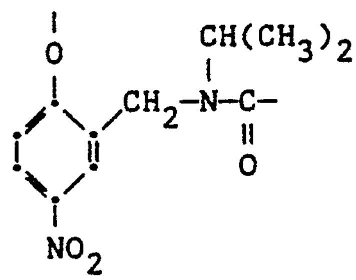

;

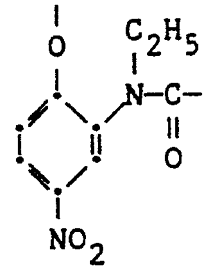

; and

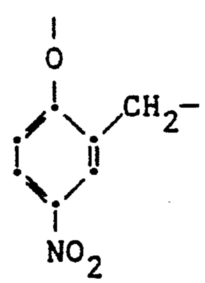

.

Other substituents can be present in the benzene ring provided they do not adversely affect the rate of coupling or cyclization to release -R.

The anchimeric compounds of group II can be made by conventional methods used to make anchimeric dye releasing couplers of, for example, U.S. Patent 3,443,940 or U.S. Patent 3,148,062.

In general the compounds of groups I and II have structures similar to those disclosed in European Patent Application 0 060 518 and U.S. Patents 3,148,062 and 3,443,940 except -R is different.

Examples of the compounds from which COUP- radicals may be formed and which are useful in both groups I and II compounds are as follows:

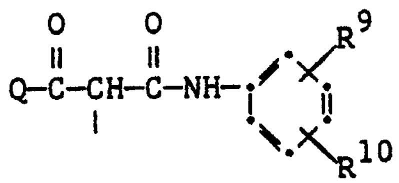

;

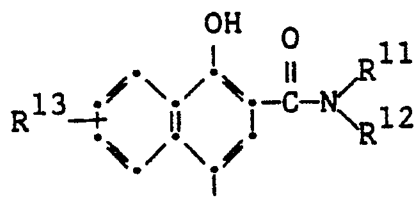

; and

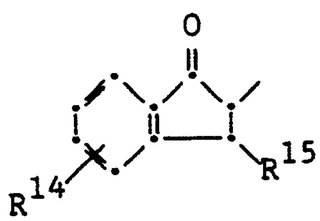

; wherein

Q represents alkyl such as methyl, t-butyl or substituted or unsubstituted aryl such as phenyl, p-methoxyphenyl;

$R^9$ and $R^{10}$ each independently represent halogen such as chloro or fluoro; hydrogen; nitro; carboxy; sulfo; substituted or unsubstituted carbonamido or sulfonamido or ureido;

$R^{11}$ and $R^{12}$ each independently represent hydrogen; alkyl such as methyl, ethyl or dodecyl; substituted or unsubstituted aryl such as tetrade-cyloxyphenyl or dicarboxyphenyl;

$R^{13}$ represents hydrogen, halogen, carboxy, sulfo, alkyl such as methyl or ethyl, sulfonamido, carbonamido, etc.;

$R^{14}$ and $R^{15}$ each independently represent hydrogen; halogen such as chloro or fluoro, carboxy, sulfo; alkyl such as methyl, ethyl or hexadecyl; substituted or unsubstituted sulfonamido or carbonamido, etc.

Preferred examples of -COUP include:

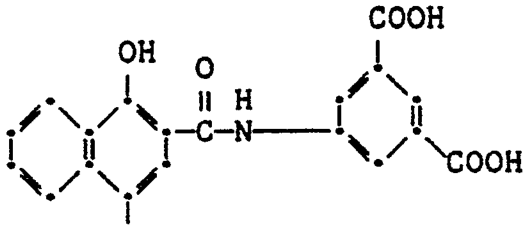

;

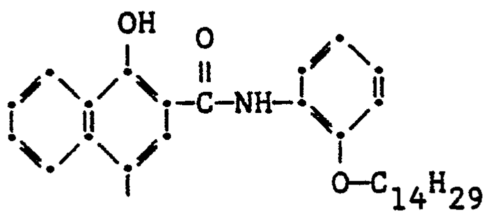

;

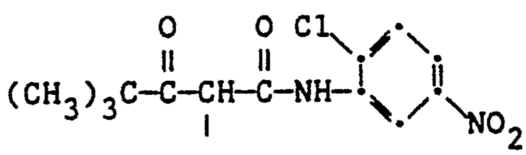

;

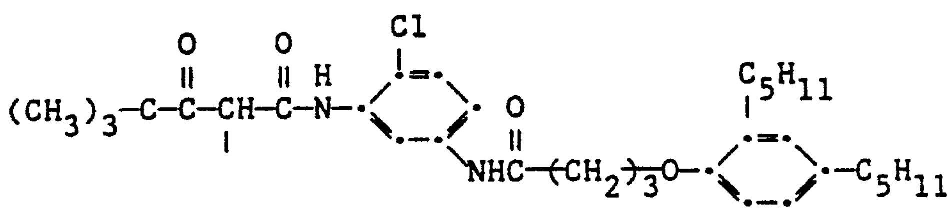

;

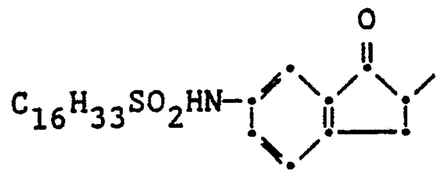 ;

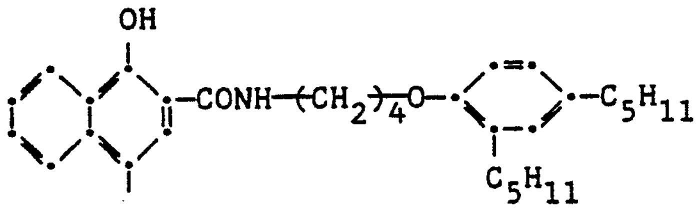 ; and

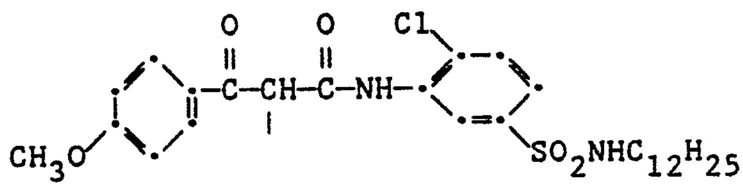 .

A portion of representative compounds according to group I are presented in Table I.

TABLE I

1) 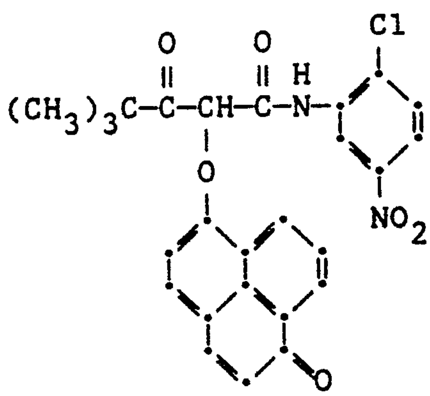 ;

2)

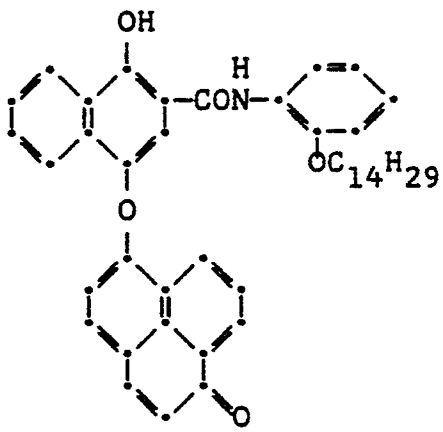

;

3)

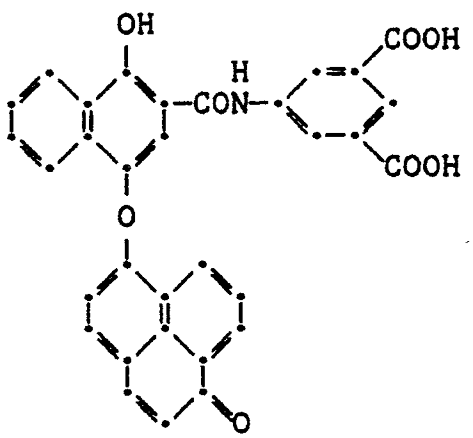

;

4)

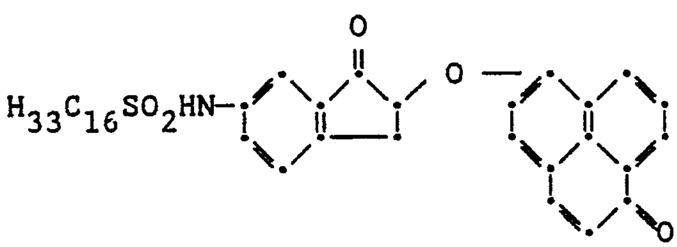

; and

5)

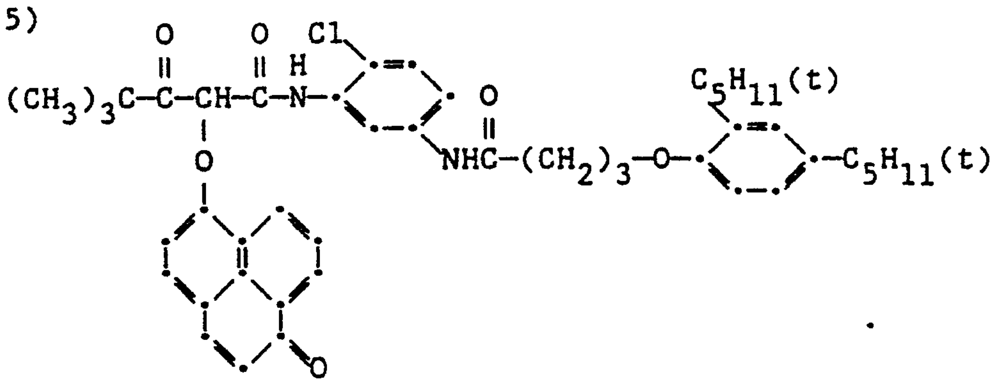

.

A portion of the representative anchimeric compounds of group II of the invention are as follows:

TABLE II

1)

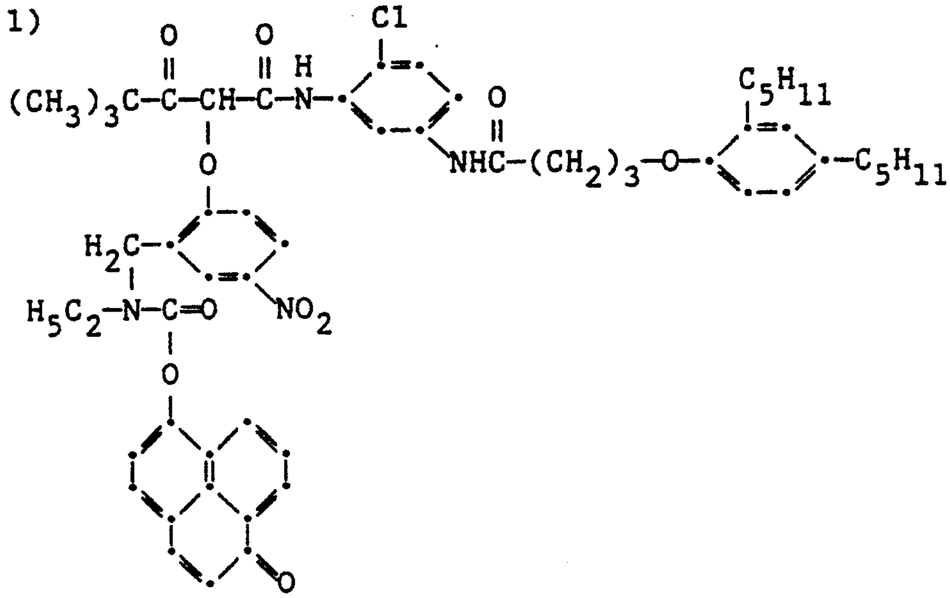

;

2)

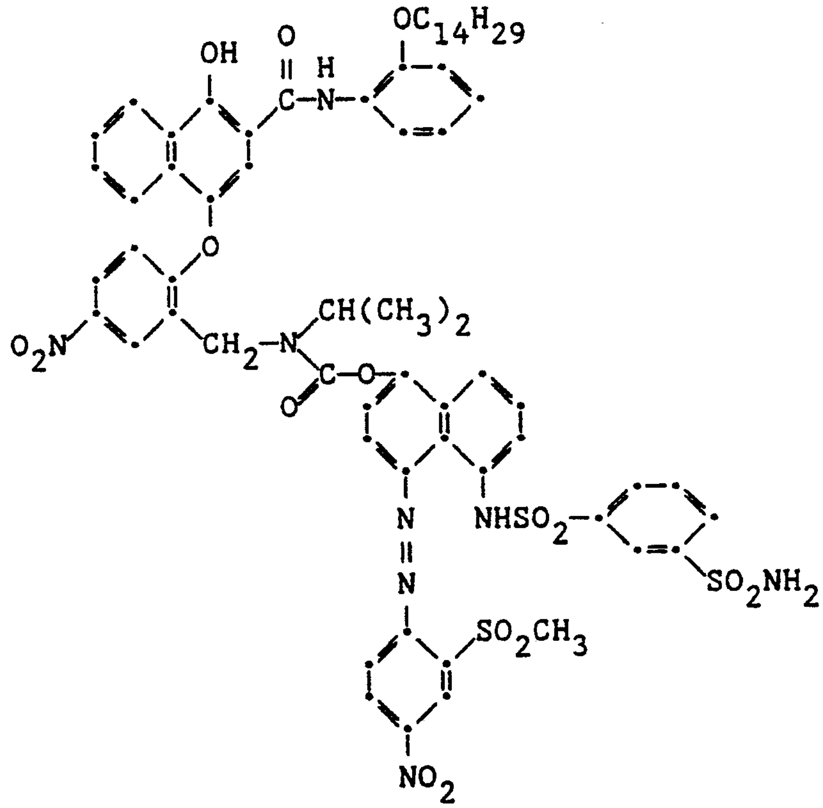

;

3)

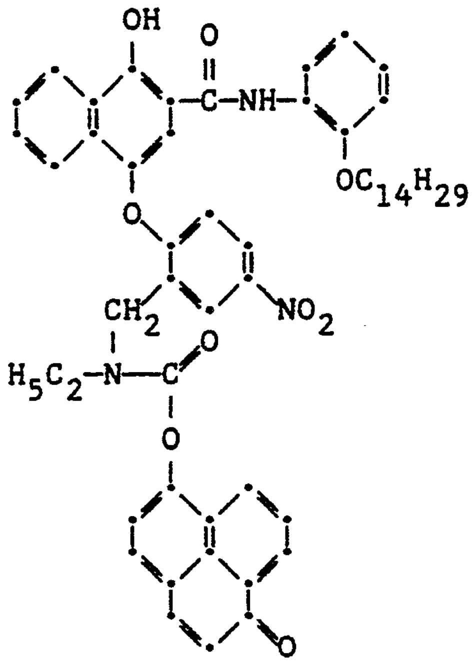

; and

4)

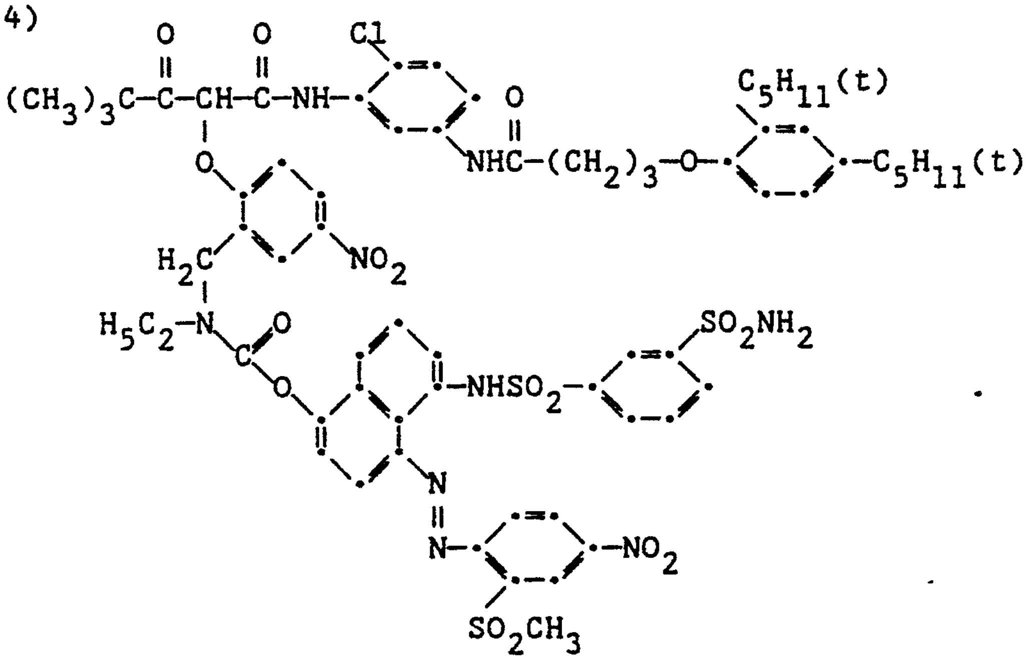

.

The hydrogen donating primary amines which are useful in this invention are those compounds designated as developers in the photographic art. Such amines include p-phenylenediamines, p-aminophenols and pyrazolidiones. A portion of representative amines are presented in Table III.

TABLE III

1) 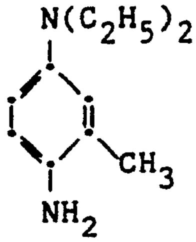 ;

2) 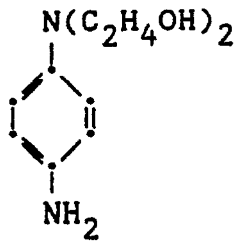 ;

3) 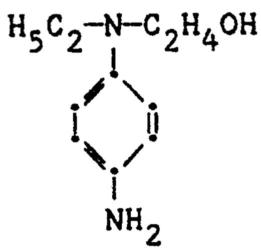 ;

4) 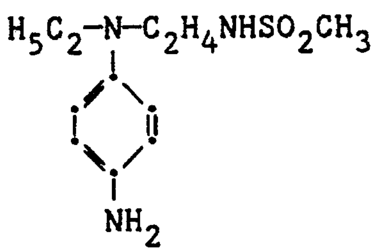 ;

5) 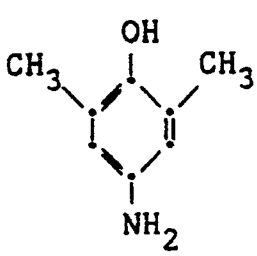 ;

6) 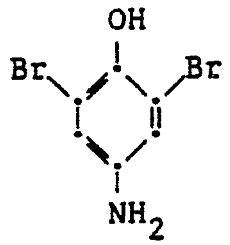 ;

7) 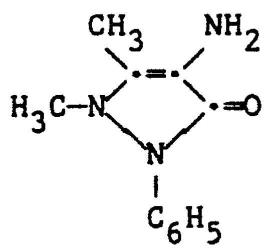 ; and

8) 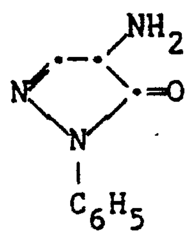 .

The reagent of the present invention provides a qualitative and quantitative determination of $H_2O_2$ in aqueous liquids. The assay can be carried out for glucose, cholesterol, ethanol, triglyceride, lipase, etc. The reagent can be used to assay biological fluids of either animals or humans, but preferably of humans. Such fluids include, but are not limited to, whole blood, plasma, sera, lymph, bile, urine, spinal fluid, sputum, perspiration and the like as well as stool secretions. It is also possible to assay fluid preparations of human or animal tissue such as skeletal muscle, heart, kidney, lungs, brains, bone marrow, skin and the like.

Peroxidases are a class of enzymes which will catalyze a reaction wherein hydrogen peroxide oxidizes another substance. The peroxidases are generally conjugated proteins containing iron porphyrin. Peroxidase occurs in horseradish, potatoes, figtree sap and turnips (plant peroxidase); in milk (lacto peroxidase); and in white blood corpuscles (verdo peroxidase); also it occurs in microorganisms and may be produced by fermentation. Certain synthetic peroxidases, such as those disclosed by Theorell and Maehly in Acta Chem. Scand, Vol. 4, pages 422-434 (1950), are also satisfactory for use in $H_2O_2$ detection systems. Less satisfactory are such materials as hemin, methemoglobin, oxyhemoglobin, hemoglobin, iron EDTA, hemochromogen, alkaline hematin, hemin derivatives, and certain other materials which demonstrate peroxidative or peroxidase-like activity, namely the ability to catalyze the oxidation of another substance by means of hydrogen peroxide and other peroxides.

Those skilled in the art of analytical chemistry will understand that the relative amounts of each component chemical included in the composition to conduct an assay for a particular analyte will depend upon the expected concentration of the analyte. Based upon such expected concentration, each component is added to obtain the necessary stoichiometric reactions.

Substantially any buffer is a suitable candidate for establishing the desired pH. Useful buffers will, of course, establish the pH in the range 5 to 10, preferably 6-9, which is conducive to the occurrence of dye development reaction while not inhibiting the reaction. Representative buffers include phosphates, borates and others reported by Good et al in Biochemistry 5, 467(1966), and Anal. Biochem., 104, 300(1980). Some

of these materials buffer the reagent composition to a range of between about 5 and 10 which is a useful pH range for detecting blood serum components using the particular indicator described herein in coupled enzymatic reaction sequences useful in the assay of glucose using glucose oxidase to produce hydrogen peroxide.

The compositions described herein may be incorporated into bibulous or absorbent analytical elements of the type well known in the art.

The reagent composition of the present invention can be incorporated into fibrous filter paper type testing materials or multilayered analytical elements of the type described in U.S. Patent 3,992,158.

The simple fibrous element can be composed of an absorbent carrier material, for example, a thin sheet of a self-supporting absorbent or bibulous material, such as filter paper or strips, which contains the analytical composition of this invention. The element can be divided into two or more discrete zones with different reagents incorporated into individual zones of the carrier material. Such elements are known in the art as test strips, diagnostic elements, dip sticks or diagnostic agents.

One useful element is disclosed in commonly assigned European Publication No. 0255087, February 3, 1988. The element comprises a water soluble polymer in which a reagent is included.

Useful absorbent carrier materials are insoluble and maintain their structural integrity when exposed to water or biological fluids such as whole blood or serum. Useful elements can be prepared from paper, porous particulate structures, porous polymeric films, cellulose, glass fibers, woven and nonwoven fabrics (synthetic and nonsynthetic) and the like. Useful materials and procedures for making such elements are well known in the art as exemplified in U.S. Patents 3,092,465; 3,802,842; 3,915,647; 3,917,453; 3,939,357; 4,248,829; 4,255,384; 4,270,920; and 4,312,834.

Preferably, the absorbent carrier material of the above analytical element contains a porous spreading zone. This zone can be self-supporting (that is, composed of a material rigid enough to maintain its integrity), but preferably it is carried on a separate support. Such a support can be any suitable dimensionally stable, and preferably, nonporous and transparent (that is, radiation transmissive) material which transmits electromagnetic radiation of a wavelength between 200 and 900 nm. A support of choice for a particular element should be compatible with the reflectance spectroscopy mode of colorimetry or fluorescence. Useful supports can be prepared from paper, polystyrene, polyesters, polycarbonates, cellulose esters and others known in the art.

The porous spreading zone can be prepared from any suitable fibrous or non-fibrous material or mixtures of either or both. The void volume and average pore size of this zone can be varied depending upon the use intended.

Useful spreading zones can be prepared using fibrous materials, either mixed with a suitable binder material or woven into a fabric, as described in U. S. Patent 4,292,272, polymeric compositions or particulate materials, for example, blush polymers as described in U. S. Patent 3,992,158, beads bound together with or without binding adhesives, as described in U. S. Patents 4,258,001 and 4,430,436 and Japanese Patent Publication 57(1982)-101760. It is desirable that the spreading zone be isotropically porous, meaning that the porosity is the same in each direction in the zone as caused by interconnected spaces or pores between particles, fibers or polymeric strands.

The dry element can have two or more discrete zones, either in the same layer or superimposed. At least one of the zones is preferably a porous spreading zone or a combination spreading/reagent zone. The other zones can be reagent zones or registration zones as those zones are known in the art, additional spreading zones, radiation-blocking or filter zones, subbing zones or barrier zones. The zones are generally in fluid contact with each other, meaning that fluids, reagents and reaction products (for example, color dyes) can pass or be transported between superposed regions of adjacent zones. In other words, when the element is contacted with fluid, all reagents of the analytical composition of this invention become mixed and can readily move within the element as a composition. Preferably a multilayer element is used in which each zone is a separately coated layer, although two or more zones can be separate areas in a single layer of the element. Besides the references noted above, suitable element components are described also in U.S. Patents 4,042,335; 4,132,528 and 4,144,306.

The preferred multilayer element is placed in use by applying to the element a sample of liquid under analysis. Typically, an element will be formed such that an applied sample will contact a spreading layer, or a combination spreading/reagent layer, prior to the reagent layer and will first contact such spreading layer at its surface furthest removed from such reagent layer. Because analytical accuracy of the present elements is not substantially diminished by variations in the volume of applied samples, sample application by hand or machine is acceptable. For reasons of convenience in detecting an analytical result, however, reasonable consistency in sample volume may be desirable.

In a typical analytical procedure using the elements, which could be manual or automated, the element

is taken from a supply roll, chip packet or other source and positioned to receive a free drop, contact spot or other form of liquid sample, such as from an appropriate dispenser. After sample application, and desirably after the liquid sample has been taken up by a spreading layer, the element is exposed to any conditioning, such as heating, humidification or the like, that may be desirable to quicken dor otherwise facilitate obtaining any test result. To avoid migration of a developed dye spot after reaction of a sample solution with the reagent composition of the invention, the reagent or registration zone or layer of the element may include a mordant such as poly(styrene-co-m + p-N-vinylbenzyl-N-benzyl-N,N-dimethyl ammonium chloride (60,40)-co-divinylbenzene (49.5; 49.5; 1).

After the analytical result is obtained as a detectable change, it is measured, usually by passing the element through a zone in which suitable apparatus for reflection or transmission spectrophotometry is provided. Such apparatus would serve to direct a beam of energy, such as light, through the support and the reagent layer. The light would then be reflected, such as from an opacifying agent in the spreading or a radiation-blocking layer in the element, back to a detecting means or would pass through the element to a detector, in the case of transmission detection. In a preferred mode, the analytical result is detected in a region of the element totally within the region in which such result is produced. Generally, electromagnetic radiation in the range of from 400 to 700 nm has been found useful for such measurements, although any radiation to which the element is permeable and which is capable of quantifying the detectable change produced in the element can be used. Various calibration techniques can be used to provide a control for the analysis. As one example, a sample of analyte standard solution can be applied adjacent to the area where the drop of sample is placed in order to permit the use of differential measurements in the analysis.

The elements of this invention can also contain one or more other addenda commonly put in the elements for various manufacturing or operational advantages. Such addenda include surfactants, buffers, solvents, hardeners and other materials known in the art.

The following examples demonstrate the use of the reagents of the invention in the determination of $H_2O_2$.

Example 1 - Assay for Glucose Oxidase Activity

This example illustrates an assay for glucose oxidase, a $H_2O_2$-generating oxidase enzyme, using Compound 1, Table II.

A dispersion of the Compound 1 was prepared as follows. Compound 1 (4 mg) was dissolved in N,N-dimethylformamide (DMF, 250 µL). A surfactant, Triton X-100 (500 µL), was added. The solution was mixed and added slowly with stirring to 25 mL of 0.05 M potassium phosphate buffer (pH 7.5).

The assay was run as follows:

A test solution was prepared from the dispersion (1 mL), 4-amino-3-methyl-N,N-diethylaniline (25 µL), glucose (0.3 mL of a 10% aqueous solution), glucose oxidase (0.1 mL buffer of 1 mg/mL solution, 39 units/mg, Type II, Sigma Chem Co), horseradish peroxidase (0.1 mL of 60 units/mL buffer solution, 210 units/mg solid, and the buffer (1.6 mL).

A control solution was prepared in the same manner, using 1.7 mL of buffer and no glucose oxidase.

Fluorescence was measured over 5 minutes (excitation 540 nm, emission 620 nm). The test solution showed an increase of 17 relative fluorescence units, while the control showed an increase of only 3 units.

Example 2 - Assay for Glucose Oxidase Activity

A dispersion of Compound 2, Table II, was prepared by dissolving Compound 2 (16 mg) in DMF (1 mL); 250 µL of this solution was mixed with Triton X-100 solution (0.5 mL) and the resulting solution added slowly to 25 mL of 0.05 M of the buffer, pH 7.5.

A test solution was prepared from the dispersion (1 mL), the amine of example 1 (25 µL), glucose (0.3 mL of an 18% aqueous solution) glucose oxidase (0.1 mL of a 1 mg/mL buffer solution), 39 units/mg), horseradish peroxidase (0.1 mL of a 60 units/mL buffer solution) and buffer (1.5 mL).

A control solution was prepared in the same manner, using 1.6 mL of buffer and no glucose oxidase.

The optical density (OD) was measured at 37°C at 635 m. The OD after 10 minutes was 3.272 for the test solution and 0.269 for the control.

Example 3 - Assay for Glucose Oxidase with Reagents Coated in a Dry Element

In this example, reagents for the detection of glucose oxidase are coated on opposite sides of a poly-(ethylene terephthalate) support.

One side of the support was coated with a layer comprising surfactant Zonyl FSN, Compound 3, Table 1, glucose, horseradish peroxidase and poly(acrylamide-co-N-vinylpyrrolidone), 90:10. The other side of the support was coated with a layer comprising surfactant Zonyl FSN, 4-amino-3-methyl-N,N-diethyl-aniline and poly(acrylamide-co-N-vinylpyrrolidone), 90:10.

A portion of this element (~ 1/cm$^2$) was added to a solution containing 3 mL of potassium phosphate buffer, 0.05M, pH 7.5 and 100 $\mu$L of glucose oxidase, 1 mg/mL buffer solution, 39 units/mg. A second portion was added to a buffer solution without glucose oxidase for a background control.

Fluorescence was then measured at excitation 540 nm and emission 620 nm. After 10 minutes, the solution containing the glucose oxidase showed a large increase in fluorescence over the background control.

## Claims

1. A reagent comprising:
   a) material having peroxidative activity and
   b) a hydrogen donating primary amine characterized in that the reagent also includes
   c) a compound selected from those having the general formula:

$COUP{-}(LINK)_n{-}R$

wherein

COUP- represents a radical that couples with an oxidized primary amine and releases -(LINK)$_n$-R;

-LINK- represents a divalent radical that undergoes intramolecular cyclization and release of -R upon release by COUP-;

n represents zero or one;

-R represents a monovalent radical that forms a detectable species in the form of a colorimetric dye or fluorescent compound upon release from -LINK- or COUP-;

wherein -R is selected from the group consisting of:

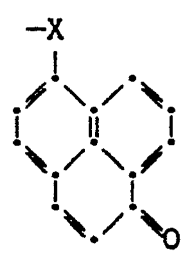 ; 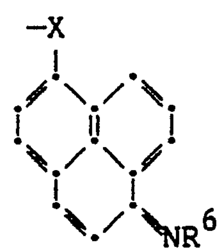 ;

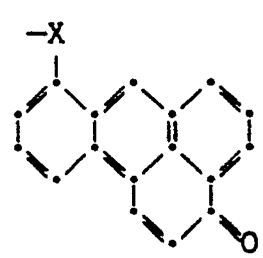 ; 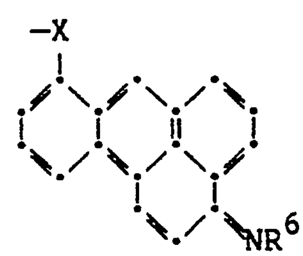 ;

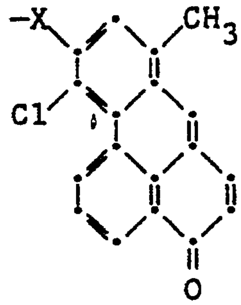 ; 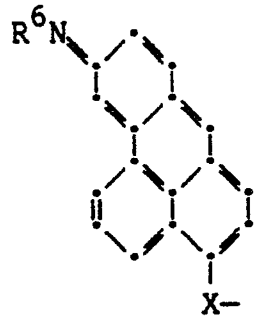 ;

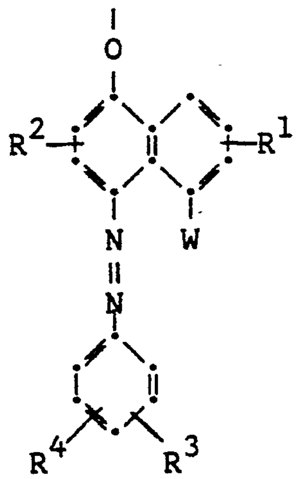 ;

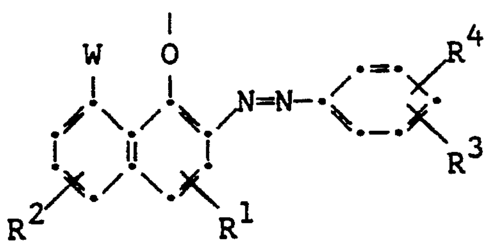 ;

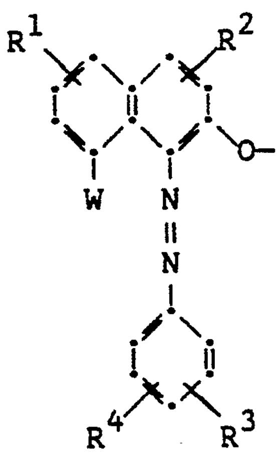 ; wherein

W represents hydrogen, halogen; hydroxy; substituted or unsubstituted carbonamido; sulfonamido; phenylsulfonyl; ureido or amino;

$R^1$ and $R^2$ each independently represent hydrogen, halogen, alkyl, alkoxy, carboxy, sulfo, cyano, nitro, carboxylic acid ester, benzoyl, carbonamido, sulfonamido, alkylsulfonyl, arylsulfonyl; and

$R^3$ and $R^4$ each independently represent halogen, nitro, sulfonamido, carbonamido, benzoyl, cyano, alkylsulfonyl, arylsulfonyl;

$R^6$ represents H, $CH_3$ or $C_2H_5$;

X represents -O-, -S- or

- $NR^5$; and

$R^5$ represents H, alkyl, cycloalkyl or aryl.

2. The reagent of claim 1 wherein the hydrogen donation primary amine is selected from the group consisting of p-phenylenediamines, p-aminophenols and pyrazolidones.

3. The reagent of claim 1 or claim 2 wherein the hydrogen donating primary amine is 4-amino-3-methyl-N,N-diethyl-aniline.

4. The reagent of any one of the preceding claims wherein COUP- is selected from the group consisting of

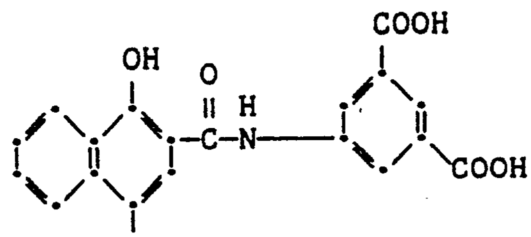

;

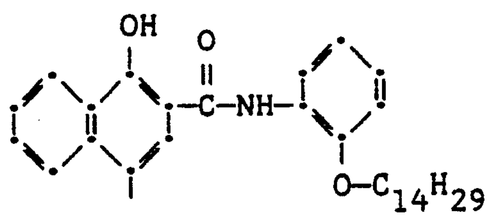

;

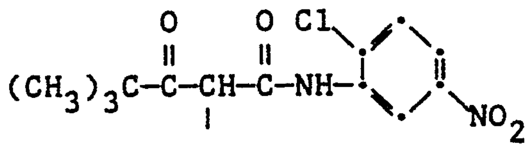

;

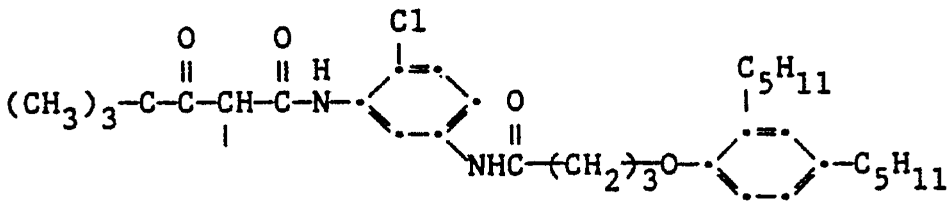

;

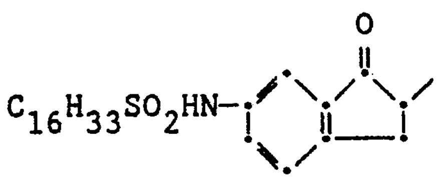

;

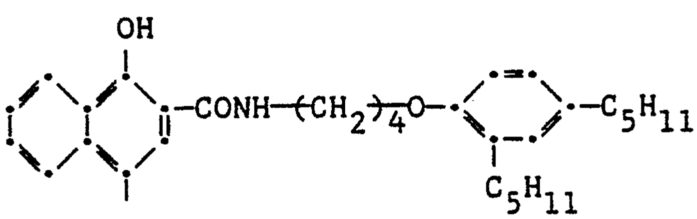

; and

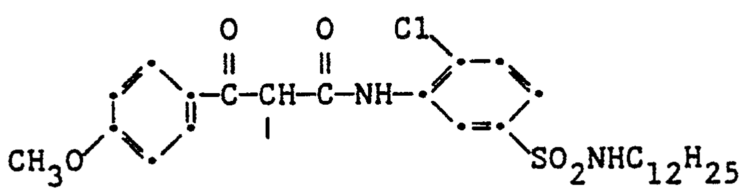

.

5. The reagent of any one of the preceding claims wherein -LINK- is selected from the group consisting of

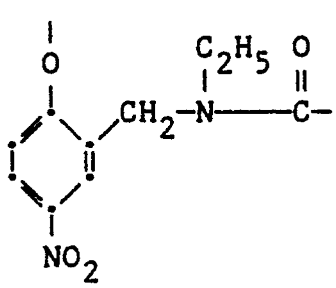

;

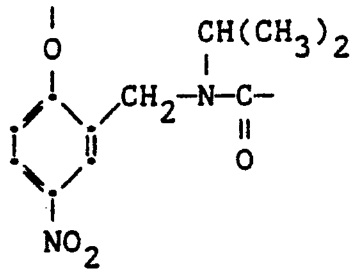

;

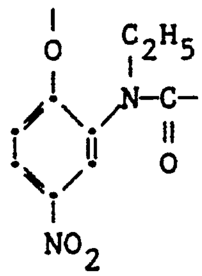

; and

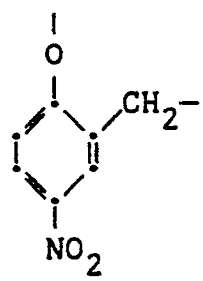

.

6. The reagent of any one of the preceding claims wherein the material having peroxidative activity is peroxidase.

7. The reagent of any one of the preceding claims wherein the COUP$\{$LINK$\}_n$R compound is selected from the group consisting of

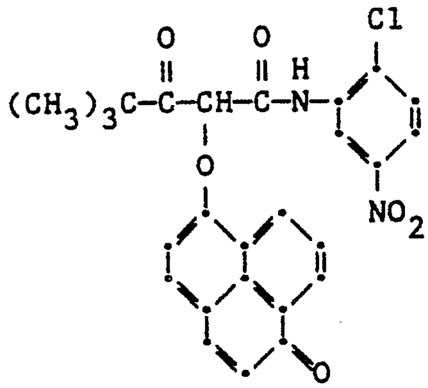

;

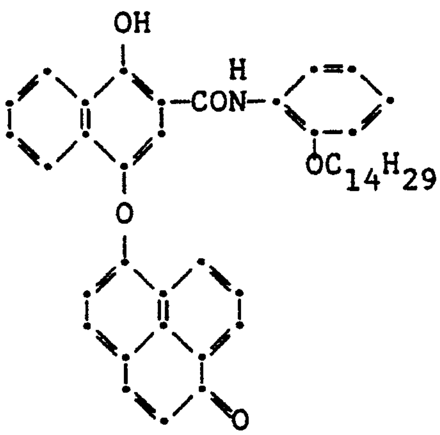

;

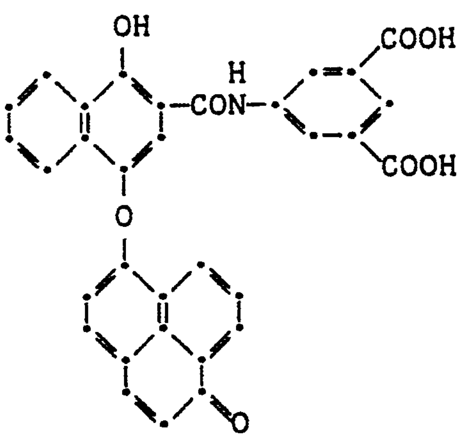

;

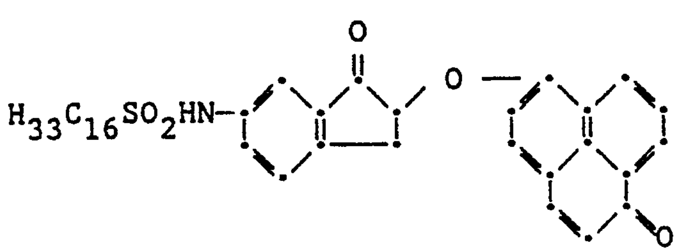

;

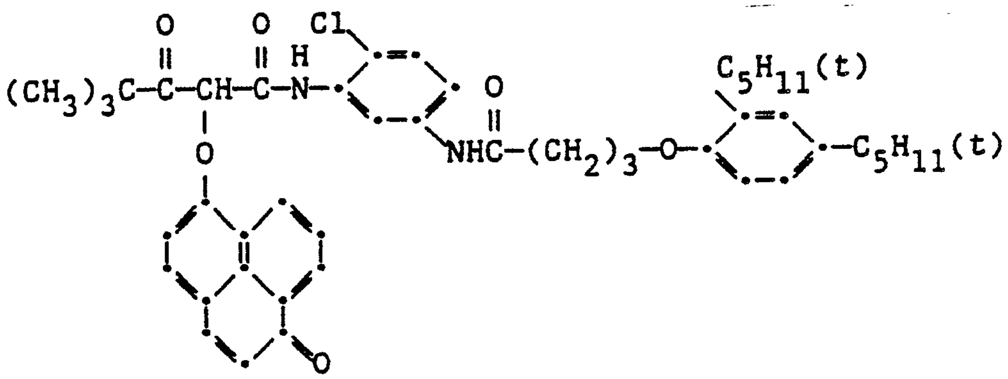
;

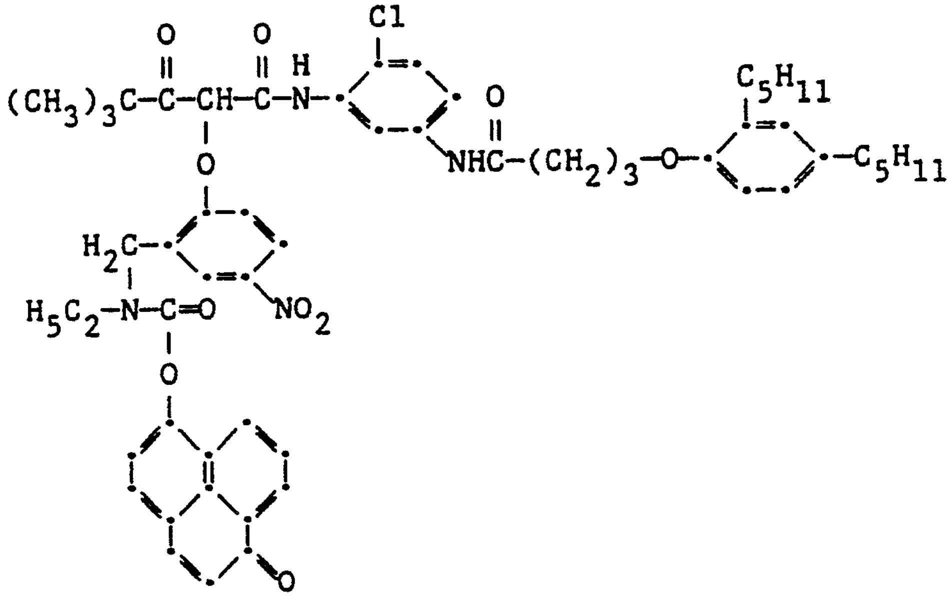
;

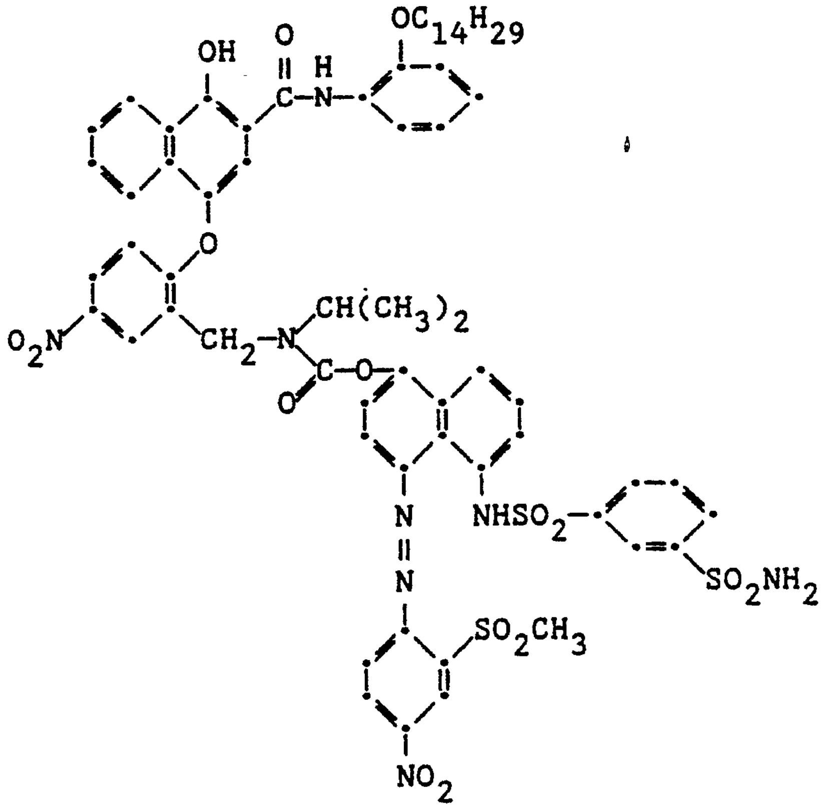

;

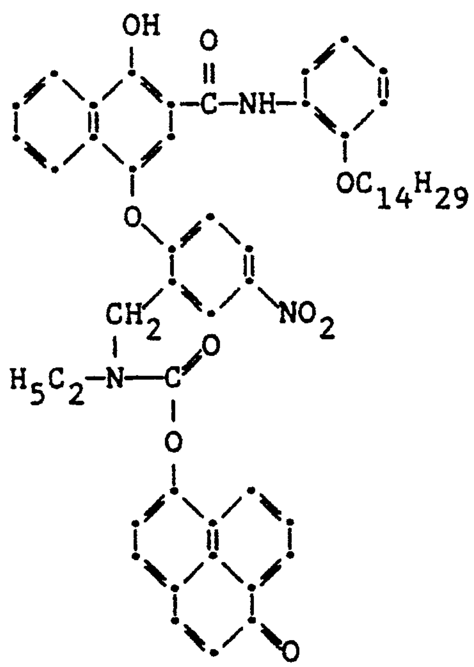

; and

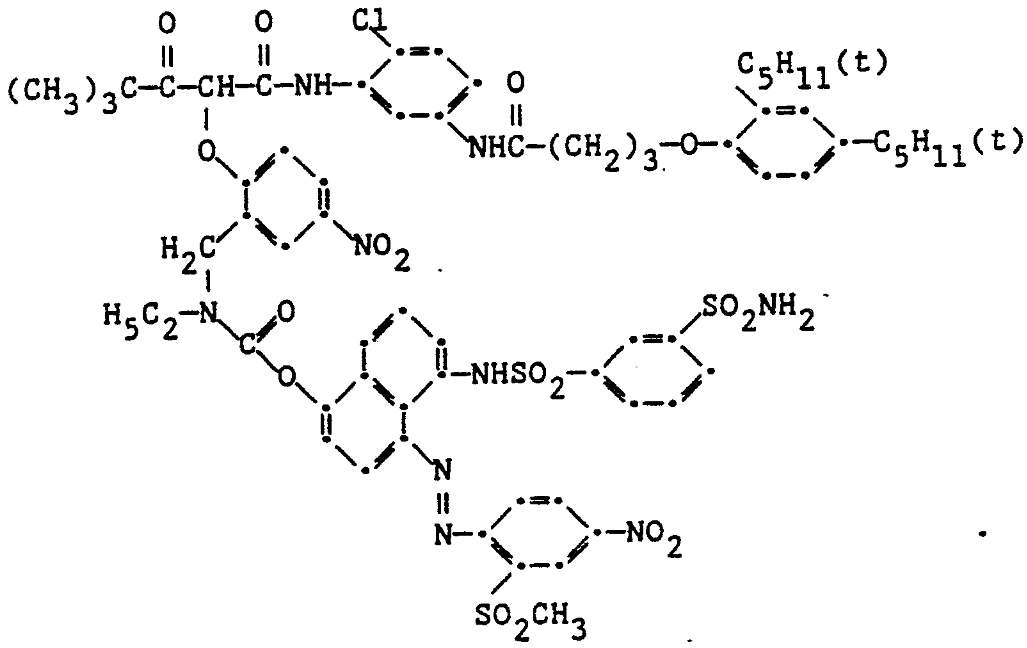

8. A method of assay for $H_2O_2$ or $H_2O_2$ generating analytes comprising the steps of

a) providing a reagent according to any one of the preceding claims;

b) providing a material capable of containing $H_2O_2$;

c) mixing an aliquot of a) with the material of b); thereby providing a color or fluorescence in the mixture; and

d) measuring the color or fluorescence.

9. An analytical element for assaying hydrogen peroxide in aqueous liquids comprising an absorbent material or a water soluble polymer containing a reagent according to any one of claims 1 to 7.

European Patent Office

# EUROPEAN SEARCH REPORT

Application Number

EP 89 30 4396

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | EP-A-0 060 518 (FUJI PHOTO FILM CO. LTD) * Page 1, lines 2-6; page 30, lines 1-11; page 31, lines 6-11 * | 1-5 | C 12 Q 1/28<br>G 03 C 7/30<br>G 01 N 33/52 |
| Y | US-A-4 248 962 (P.T.S. LAU) * Column 10, lines 20-25; column 11, lines 15-19,40-45; column 12, lines 60-68; column 15, lines 1-30 * | 1-5 | |
| A | DE-A-1 955 901 (AGFA-GEVAERT AG) * Page 3, lines 25-30; page 9, lines 28-32; page 10, lines 1-19 * | 1-3 | |
| A | EP-A-0 232 130 (EASTMAN KODAK CO.) * Page 11, lines 29-39; claims * | 1-5 | |
| A | EP-A-0 231 125 (EASTMAN KODAK CO.) * Pages 5-6 * | 1-5 | |
| A | EP-A-0 231 126 (EASTMAN KODAK CO.) * Claims 1-5 * | 1-5 | TECHNICAL FIELDS SEARCHED (Int. Cl.4)<br>C 12 Q 1/00<br>G 03 C 7/00<br>G 01 N 33/52 |

The present search report has been drawn up for all claims

| Place of search | Date of completion of the search | Examiner |
|---|---|---|
| THE HAGUE | 04-08-1989 | DUPART J-M.B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document